# EUROPEAN PATENT SPECIFICATION

(11) **EP 4 370 028 B1**
(45) Date of publication and mention of the grant of the patent: **01.07.2026**
(21) Application number: 22748039.9
(22) Date of filing: 12.07.2022
(51) Int. Cl.: A61B 5/11, G01C 22/00, A61B 5/287, A61N 1/365

(54) **MEDICAL DEVICE AND METHOD FOR DETERMINING THE NUMBER OF STEPS USING SUCH MEDICAL DEVICE**
MEDIZINISCHE VORRICHTUNG UND VERFAHREN ZUR BESTIMMUNG DER ANZAHL VON SCHRITTEN UNTER VERWENDUNG SOLCH EINER MEDIZINISCHEN VORRICHTUNG
DISPOSITIF MÉDICAL ET PROCÉDÉ POUR DÉTERMINER LE NOMBRE D'ÉTAPES UTILISANT UN TEL DISPOSITIF MÉDICAL

(30) Priority: 14.07.2021 US 202163221522 P; 23.08.2021 EP 21192601
(43) Date of publication of application: 22.05.2024
(73) Proprietor: BIOTRONIK SE & Co. KG, 12359 Berlin (DE)
(72) Inventor: PAMELA SHAMSIE VICTORIA, Riahi, Portland, Oregon 97232 (US); KIBLER, Andrew B., Lake Oswego, Oregon 97035 (US)
(74) Representative: Biotronik Corporate Services SE
(86) International application number: PCT/EP2022/069420
(87) International publication number: WO 2023/285440

(56) References cited:
- JP-A- H1 142 220
- TW-A- 201 413 220
- US-A1- 2008 255 626
- US-A1- 2016 209 232
- US-A1- 2017 000 384
- US-A1- 2018 035 920
- BERBAKOV LAZAR ET AL: "Smart-phone application for autonomous indoor positioning", 2015 IEEE INTERNATIONAL INSTRUMENTATION AND MEASUREMENT TECHNOLOGY CONFERENCE (I2MTC) PROCEEDINGS, IEEE, 11 May 2015 (2015-05-11), pages 670 - 674, XP033171117, DOI: 10.1109/I2MTC.2015.7151348

## Description

The invention is directed to a medical device for implantation within a patient's body. Such medical device is usually configured to monitor health conditions of a patient and/or deliver a therapy to a patient. The invention is further directed to a method for determining the number of steps made by a patient.

An active or passive medical device, for example, a pacemaker (with leads), a BioMonitor, an Implantable Leadless Pacer (ILP), an Implantable Leadless Pressure Sensor (ILPS), an Implantable Cardiac Defibrillator (ICD) or a Shockbox, a device that delivers spinal cord stimulation (SCS), deep brain stimulation (DBS) or neurostimulation or a device that delivers one or more therapeutic substances, e.g. a drug pump, contains sensors that collect physiological signals in order to monitor the health status of the patient and/or to deliver a therapy to the patient.

The quality of life of a patient suffering from chronic conditions such as chronic pain or congestive heart failure (CHF) is highly related to the patient's physical activity. For example, it is known that there is a close link between inactivity and depression. Therefore, therapeutic success for patients suffering from chronic conditions is usually assessed by pain level questionnaires along with perceived quality of life, analgesic use, ability to work, activities of daily living, functional capacity, psychological status and sleep quality. However, such assessments are all self-reported and performed during follow-up visits. Thus, such data are inherently qualitative and based on subjective opinion and bias of the patient. Further, such data can only be recorded during visits, wherein important information between visits may easily be left out.

The problem of recording patient data, despite standardized scales and questionnaires, is a recurring but critical issue in the design of clinical trials to evaluate the outcomes of treatment of patient's suffering from chronic conditions. There is a clear common desire among physicians and researchers to objectively quantify a patient's condition, whether to appropriately adjust treatment parameters or to assess treatment efficiency.

Determining a patient's physical activity level through step count is an objective measure of quality of life for patients with a variety of chronic conditions. For example, for patients suffering from chronic pain, greater walking ability enables them to perform important activities of daily living, such as grocery shopping, household tasks and visits to the park. Therefore, measuring the improvement of the physical activity as a therapy outcome is highly desirable.

Devices and methods for step counting according to the state of the art are for instance described in BERBAKOV LAZAR ET AL: "Smart-phone application for autonomous indoor positioning", 2015 IEEE INTERNATIONAL INSTRUMENTATION AND MEASUREMENT TECHNOLOGY CONFERENCE (I2MTC) PROCEEDINGS, IEEE, 11 May 2015, pages 670-674, DOI: 10.1109/ I2MTC.2015.7151348, as well as JPH1142220A, US2016/0209232A1, TW201413220A, US2017/0000384A1, US2008/0255626A1 and US2018/0035920A1.

Devices and corresponding algorithms for step counting are well known in the state of the art, e.g. fitness trackers. However, the devices are often expensive and cannot be integrated into medical devices. In addition, the algorithms are too complex, require too much power and/or processing capacity to be implemented in medical devices with strict size and power constraints. At the same time, sensors such as accelerometers are often already integrated within the medical devices and may be used to provide data about the physical activity of a patient.

Accordingly, it is desirable to use this data gathered by those sensors in order to determine the physical activity. More specifically, it is desirable to use the data from an accelerometer unit of a medical device for step counting. Therefore, it is an object to provide an implantable medical device and a corresponding method for step counting.

### SUMMARY OF THE INVENTION

According to the invention there are provided a medical device with the features of claim 1, by a method with the features of claim 9, a computer program product with the features of claim 14 and a computer readable data carrier with the features of claim 15.

### SUMMARY OF THE DISCLOSURE

The medical device is any device which is configured to be implanted partly or fully within a patient's body or fixedly mounted or wearable at the patient's body and configured to monitor the health condition of a patient and/or to deliver a therapy to the patient. A so-called fitness tracker is regarded as medical device as it monitors the fitness as a health condition of the patient. Further, as an implanted device the medical device is generally fixed within the patient's body, the medical device may carry out no or only very small relative movement with regard to the patient's upper part of the body (chest, back, neck, head). Particularly, the medical device may be, for example, a pacemaker, ILP, ICD, Shockbox, device that delivers SCS, or a BioMonitor.

The medical device comprises the accelerometer unit and the processor which are electrically interconnected. This means, inter alia, that they are capable to exchange data.

For the patient's body two orthogonal axes are defined, wherein the first orthogonal axis is given by the craniocaudal axis of the patient's body and the second orthogonal axis is given by the dorsoventral axis of the patient's body for a patient standing in its upright position. However, for those patients who may have assumed a curved posture in their 'upright' position, for example due to a medical condition, the axis similar to the dorsoventral axis, i.e. the second orthogonal axis, is further defined to be parallel to the ground or from behind the patient to the front of the patient, wherein the second orthogonal axis may be further characterized as forming the axis of motion along which the patient moves while walking. Additionally, in the 'upright' position, the craniocaudal axis, i.e. the first orthogonal axis, is further defined to be perpendicular to the ground or runs from the toes to the head of the patient (axis similar to the craniocaudal axis). Additionally, the "similar" axes further comprise axes that are slightly tilted (by some degrees) with regard to the above defined first and second orthogonal axes. Therefore, during each step the patient's body performs a movement along the first orthogonal axis as well as the second orthogonal axis, wherein forward movement of the patient is along the second orthogonal axis and with the patient's body raising and lowering in the direction of the first orthogonal axis during each step.

The accelerometer unit, which is provided in order to gather the acceleration data caused by the above described movement of the patient's body, i.e. of the medical device, may comprise a 2-dimensional accelerometer sensor, for example one 2-dimensional accelerometer or several 1- or 2-dimensional accelerometers, such as a piezo electric and/or micro-electro mechanical (MEMs) accelerometer and/or mechanical accelerometer and/or gravimeter or any combination of these sensors. A respective 3-dimensional accelerometer may be used, as well. The term at least 2-dimensional means that the acceleration unit is configured to determine at least 2-dimensional proper acceleration data along sensitive axes corresponding to the above-defined first and second orthogonal axes of the patient's body. Accordingly, the acceleration data determined by the acceleration unit always comprise two data components, namely the acceleration along the first orthogonal axis of the patient and the acceleration along the second orthogonal axis of the patient. Proper acceleration is the acceleration (the rate of change of velocity) of the unit in its own instantaneous rest frame. For example, an accelerometer at rest on the surface of the Earth will measure an acceleration into the direction of gravity due to Earth's gravity, straight upwards (by definition) of g ≈ 9.81 m/s² because the Earth's surface exerts a normal force upwards relative to the local inertial frame (the frame of a freely falling object near the surface). By contrast, accelerometers in free fall (falling toward the center of the Earth at a rate of about 9.81 m/s²) will measure zero. Usually, the acceleration is quantified in the SI unit m/s² or in terms of standard gravity (multiples of g).

The processor is regarded as a functional unit of the medical device, that interprets and executes instructions comprising an instruction control unit and an arithmetic and logic unit. The processor may comprise a microprocessor, a controller, a digital signal processor (DSP), an application specific integrated circuit (ASIC), a field-programmable gate array (FPGA), discrete logic circuitry or any combination thereof. The processor may process the (raw/fresh/new) acceleration data received from the accelerometer unit directly or the acceleration data received from a data memory unit.

The medical device may comprise further modules/units such as the data memory unit, a power supply such as a battery, at least one signal generator for generating, for example, electrical or electromagnetically therapy signals in order to provide the therapy to the patient, a sender module for sending data to an external unit such as a remote computer or Programmer and/or a transceiver module for bi-directionally exchanging data with such remote computer or Programmer. The sender transceiver module, the power supply, the at least one sensor and/or the signal generator may be electrically connected to the processor. All above mentioned modules/units may be modules/units separate from the processor or may be at least partly integrated within the processor of the medical device. The sender module and/or the transceiver module may communicate wirelessly with the remote computer or Programmer, for example using electromagnetic waves, for example Bluetooth, WLAN, ZigBee, NFC, Wibree or WiMAX in the radio frequency region, or IrDA or free-space optical communication (FSO) in the infrared or optical frequency region. Communication by wire (electrical and/or optical communication) may be possible, as well. The remote computer is a functional unit that can perform substantial computations, including numerous arithmetic operations and logic operations without human intervention, such as, for example, a personal mobile device (PMD), a desktop computer, a server computer, clusters/warehouse scale computer or embedded system.

The data memory unit of the medical device may include any volatile, non-volatile, magnetic, or electrical media, such as a random-access memory (RAM), read-only memory (ROM), non-volatile RAM (NVRAM), electrically-erasable programmable ROM (EEPROM), flash memory, or any other memory device. The data memory unit may store the measured (raw) acceleration data determined by the acceleration unit.

In one embodiment, accelerometer unit is implanted within or fixedly mounted to the patient's body as it is contained within the medical device. Further, the accelerometer unit may be calibrated in such a way that acceleration values/data of the patient along the first orthogonal axis as well as along the second orthogonal axis are gathered.

It is important that the accelerometer unit continuously detects acceleration data along the first orthogonal axis as well as along the second orthogonal axis as defined above, for example while the patient is walking. In some operation modes of the medical device the accelerometer unit may be switched off for power saving. During such time the processor does not perform the method steps for accelerometer data processing and for step count determination. Alternatively or additionally, the acceleration data processing and step counting may be paused as long as no sufficient patient activity is observed.

Thus, the medical device is implanted within or mounted on body parts of the patient's body such as the thorax or the pelvis, which have a generally fixed orientation to the patient's upper body, wherein the medical device is permanently or temporarily attached to the patient's body, for example by suturing.

In other words, in one embodiment to allow the medical device to continuously over time determine at least 2-dimensional acceleration data along the above-defined first orthogonal axis and the second orthogonal axis of the patient's body suitable for step counting using the processor operation, the accelerometer unit is fixed with regard to these axes, i.e. does not or only very slightly rotate or otherwise move in relation to the patient's body even if the patient is moving. The axes of the accelerometer unit may have a fixed orientation in relation to both above-defined axes of the patient's body. If the orientation of the axes of the accelerometer unit with regard to the patient's body is known, the acceleration component with regard to the first orthogonal axis and to the second orthogonal axis, respectively, may be determined and used for further processing in order to count steps of the patient.

Further, the acceleration data is determined continuously for each point in time along the first orthogonal axis as well as along the second orthogonal axis, wherein the frequency of measurements may be determined by the frequency of the clock signal of the processor. Consequently, two data pairs are created for each time point, a first data pair consisting of the time information and the corresponding acceleration date referring to the patient's movement along the first orthogonal axis and a second data pair consisting of the time information and the corresponding acceleration data referring to the patient's movement along the second orthogonal axis. Alternatively, a data triple may be determined for each time point containing the time information, the acceleration date along the first orthogonal axis and the acceleration date along the second orthogonal axis.

In one embodiment the detection of the acceleration data may only be started if the processor reveals that the state of the patient transitions from a rest state into an active state or if the step counting or accelerometer unit is activated or enabled as indicated above. In one embodiment, it is ensured by checking a pre-defined condition whether the patient is standing in its upright position. Obviously, the determination of the acceleration data may be stopped once the processor reveals that the patient is in a rest state or the patient is lying, for example by assessing accelerometer data of three orthogonal axes. The rest state may be identified using the movement intensity of the patient. If there is no movement or very low movement detected using the accelerometer unit, i.e. an average accelerometer signal amplitude equal to or below a pre-defined activity threshold it is assumed that the patient is at rest. The first time interval is a time interval with a start point and an end point in which the processor receives the acceleration data along the first axis and the second axis and for which data the processor performs the inventive step counting algorithm. The first time interval may cover, for example, a plurality of minutes or hours, the latter, e.g., if the patient goes hiking.

The acceleration data is typically perceived as an electrical signal proportional to the actual acceleration of the patient's body.

To execute the step counting algorithm, the processor is basically configured to simple operations, which, in particular, consume little processor capacity and consequently power of the power supply. Thus, most of the operations are additions, subtractions and the comparisons with pre-defined threshold values. Due to the simplicity of the algorithm, the corresponding parts of the medical device such as the processor and the accelerometer unit may also be designed to be correspondingly small. Additionally, due to the simplicity of the algorithm, it has a high computing speed.

After the processor receives acceleration data / a group of acceleration data from the accelerometer unit determined within the first time interval, these acceleration data are processed in order to determine the number of steps made by a patient within said first time interval. Some pre-processing of the data may be provided by the accelerometer unit. Additionally, the signals may be digitized by an A/D converter of the accelerometer unit or the processor prior further processing.

After that, the processor initially applies a bandpass filter with a frequency range (or cut-off frequencies ranging) between 0.001 Hz and 100 Hz to said acceleration data or group of acceleration data perceived within said first time interval. In one embodiment, the high pass cut off may be in the range of 0.001 Hz to 0.5 Hz and the low pass cut off may be in the range of 3 Hz to 100 Hz. In one embodiment, the frequency range is between 0.25 Hz and 6 Hz. Since the medical device is a device worn by ill rather than trained people, especially by patients suffering from chronic diseases, such bandpass filter in the specified frequency range is perfectly adapted to the expected motion dynamics of such people and removes signal noise.

Then, data processing continues with summing of the filtered acceleration data of the first orthogonal axis and the second orthogonal axis discretely for each point in time and smoothing said filtered acceleration data by calculating a moving average using a moving average window of a pre-defined width. Both data processing steps may be applied in any order, but due to calculation speed first summing and then smoothing of the data may be preferred.

By the summing step the step characteristic of the data is emphasized because usually the forward and backward movement of the patient during walking along the second orthogonal axis is in phase with the raising and lowering (upward and downward) movement of the patient's body along the first orthogonal axis.

Further, the filtered (and, if applicable, summed) acceleration data is smoothed by calculating a moving average (boxcar filter) using a moving average window of a pre-defined width. In this step, a simple or weighted moving average is calculated for all acceleration data within the window of the pre-defined width. In one embodiment, the width of the moving average window is between 0.1 seconds and 0.6 seconds, for example 0.3 seconds. Again, the width of the average window is adapted to the expected motion dynamic of the patient, i.e. a person suffering from illness. The smoothing step smooths the acceleration data without removal of the step-caused characteristics of the signal.

After application of the filtering and the smoothing steps, the first derivative of these acceleration data is determined. At zero crossings of the derived derivative function, the extreme points of the smoothed acceleration data, the so-called local extrema, are determined. At this point, no differentiation is made between the local extrema of the acceleration data and significant extrema, so that each extremum is considered as a local extremum. It is obvious that there may be multiple local extrema within a time interval which belongs to one walking step of the patient. Therefore, in the next method step, the processor is adapted to identify those extrema out of the local extrema, i.e. the significant extrema, eligible for step counting, based on at least one pre-defined condition. However, in this step it may be derived that all local extrema determined in the previous step may be significant extrema eligible for step counting. Usually, this step picks a subgroup of local extrema of all local extrema within the first time interval being significant extrema. Ideally, each step is characterized by one significant minimum and one significant maximum. One step may be counted from one significant minimum to the directly neighboring significant minimum (if going forth in time) in the meantime passing a significant maximum.

The number of steps may then be stored in the data memory of the medical device and/or transferred to an external computer, a Programmer or the like as mentioned above in order to be assessed by a health care provider (HCP).

As already mentioned above, the processor is configured to determine the group of "significant" extrema from the plurality of local extrema which is eligible for step counting. Accordingly, the "significant" extrema should rather read significant local extrema, but in the following "local" is omitted. There are various possibilities for this method step, some of which are described below, wherein in each method step a number of the local extrema may be excluded, so that only those local extrema eligible for the step counting, i.e. the significant extrema, remain and forming the base for patient's step counting. These method steps are referred to as identification steps in the following. The identification steps provide the "pre-defined condition" mentioned above on which the significant extrema are identified. In particular, because some of the identification steps described below can also be performed as final or checking steps, i.e. after at least some significant extrema eligible for step counting have been determined, the steps are at least partially applicable to significant extrema as well. Further, depending on the usage and location of the medical device, it may make sense to combine two or more of these steps, wherein the order of operation may be decisive.

The identification steps for determining the significant extrema contain the instruction that "one of" the respective, at least two local extrema is not regarded as a significant extremum. Generally, the other one or two local extrema may be chosen as significant extrema (if they are not excluded due to another identification step. Accordingly, there is the question which one of the at least two local extrema is chosen as being a not significant - i.e. an insignificant - extremum. For determining an insignificant extremum a different identification step or other criteria may be used, for example the extremum with the highest (or lowest) acceleration value or the extremum which is first (last) in time. In one embodiment, the following identification steps may be applied to all identified local (and significant) extrema prior determining the step count within the first time interval.

Thus, in one embodiment the processor is configured to determine a peak-to-peak amplitude of two consecutive local extrema. In a first identification step, one of the two consecutive local extrema (including significant local extrema) is not regarded as a significant extremum eligible for step counting if the determined peak-to-peak amplitude is less than or equal to a pre-defined first threshold. The peak-to-peak amplitude is obtained by subtracting one acceleration value from the second acceleration value of the consecutive extrema and determining the absolute value of the result of the subtraction. Assessing the peak-to-peak amplitudes of two consecutive local extrema allows to discard those extrema which are based on a noisy signal. In one embodiment, the first threshold may be in the range of 0.01 - 1,000, e.g. 50.

Further, in one embodiment the processor is configured to determine the polarity of two consecutive local extrema or significant extrema. One of the two extrema is not regarded as a significant extremum eligible for step counting if the acceleration data of these extrema have the same polarity. The polarity of an extremum refers to the algebraic sign of the respective acceleration value, i.e. whether the acceleration date of the extremum is positive or negative. Extrema which have an acceleration date zero may not be regarded as a significant extremum eligible for step counting. This identification step leads to removal of extrema based on a noisy signal, as well. In another embodiment, the processor is configured to determine the polarity of three consecutive local extrema or significant extrema. In this embodiment, at least one of the three extrema is not regarded as significant extremum eligible for step counting if the polarity order is not "-", "+", "-" or "+", "-", "+".

In one embodiment the processor is configured to determine a time difference between two consecutive local or significant extrema, and wherein one of the two extrema is not regarded as a significant extremum eligible for step counting if the determined time difference is greater than or equal to a pre-defined second threshold as another identification step. If two consecutive extrema are considered, the kind of the extrema, i.e. whether the first extremum represents a local minimum or a local maximum, may indicate whether the foot is being raised or lowered. Thus, depending on the kind of the extrema used for the comparison with the second threshold, the second threshold may be chosen individually for foot raising or lowering. For example, the second threshold may be in the range from 0.001 to 2 seconds, e.g. 1 second. This identification step tries to remove local extrema which do not refer to full steps and therefore may poison statistics.

In one embodiment the processor is configured to determine a time difference between a local maximum or significant maximum and a consecutive local minimum or significant minimum. However, here one of the minimum and the maximum is not regarded as a significant minimum or significant maximum eligible for step counting, respectively, if the time difference is less than or equal to a pre-defined third threshold as another identification step. As in this case a minimum is following a maximum, this identification step refers to foot lowering. This identification step is based on the finding, that during walking, the foot is usually lowered much faster than it is raised, a certain amount of time is still required for lowering the foot. If this time is faster than the third threshold, it is assumed that one of the extrema is caused by a noisy signal. In one embodiment, the third threshold may be equal to or lower than 0.5 seconds, for example 0.1 seconds.

In one embodiment the processor is configured to determine the peak-to-peak amplitudes of each two consecutive of at least three consecutive extrema, wherein the processor is configured to sum said determined peak-to-peak amplitudes, wherein at least one of the at least three consecutive extrema is not regarded as a significant extremum eligible for step counting if the sum is less than or equal to a pre-defined fourth threshold. This operation may be suitable to be performed as a final, checking identification step. The determination of the peak-to-peak amplitude for each two consecutive extrema is calculated as indicated above. By summing the determined peak-to-peak amplitudes and comparing them with the fourth threshold, it is possible to determine whether a step is actually present for supposedly eligible extrema for step counting. As the magnitude of the acceleration data highly depends on the location of the medical device within or at the patient's body, in one embodiment the fourth threshold may be chosen in the range from 0.1 m/s² to 1000 m/s².

After application of one or more identification steps the processor is configured to count the number of steps within the predefined first time interval as indicated above using the significant extrema which are all local extrema or a subgroup of the local extrema. In one embodiment this number may be an intermediate result, only, as explained in the following.

In one embodiment the processor is configured to discard a counted step if it is not preceded by a pre-defined first number of steps and/or followed by a pre-defined second number of steps within a predefined second time interval. The second time interval, which is shorter than the first time interval, may be a fraction of the first time interval, so that, for example, if the first time interval may be 30 seconds long, only the first 5 seconds may be regarded as the second time interval. The pre-defined second time interval is preferably at the beginning or at the end of the first time interval. Thus, this identification step makes sure that steps are only counted if a reasonable number of steps is identified as a walking activity. It is thereby avoided that single steps are counted likely due to noise or not relevant to overall walking level. The predefined first number may be, for example, in the range 0-10 (e.g. 2) and the second number, for example, in the range 1-10 (e.g. 2) Further, the second time interval may be e.g. in the range 0.1-3 seconds In this embodiment, the processing unit is configured to determine the final number of steps from the number counted from the significant extrema minus the discarded number of steps.

In one embodiment the width of the moving average window is between 0.1 and 0.6 seconds. In one embodiment the processor determines peak-to-peak amplitudes of two consecutive local extrema or significant extrema, wherein one of the two consecutive local extrema or significant extrema is not regarded as a significant extremum eligible for step counting if the determined amplitude is less than or equal to a pre-defined first threshold.

In one embodiment the processor determines the polarity of two consecutive local extrema or significant extrema, and wherein one of the two extrema is not regarded as a significant extremum eligible for step counting if the acceleration data of these extrema have the same polarity.

In one embodiment the processor determines a time difference between two consecutive local or significant extrema, and wherein one of the two extrema is not regarded as a significant extremum eligible for step counting if the determined time difference is greater than or equal to a pre-defined second threshold.

In one embodiment the processor determines a time difference between a local maximum or significant maximum and a consecutive local minimum or significant minimum, and wherein one of the minimum and the maximum is not regarded as a significant minimum or significant maximum eligible for step counting, respectively, if the time difference is less than or equal to a pre-defined third threshold.

In one embodiment the processor determines the peak-to-peak amplitudes of each two consecutive of at least three consecutive extrema, wherein the processor sums said determined peak-to-peak amplitudes, and wherein at least one of the at least three consecutive extrema is not regarded as a significant extremum (SE) eligible for step counting if the summed amplitude is less than a pre-defined fourth threshold.

In one embodiment the first to fourth thresholds, the first and second time interval and/or the first and second number of steps may be initially stored in the data memory of the medical device and/or may be provided by a Programmer used with the medical device. By the Programmer, the thresholds and time intervals may be adapted to the respective patient's needs.

In one embodiment the processor discards a counted step if it is not preceded by a pre-defined first number of steps and/or followed by a pre-defined second number of steps within a predefined second time interval as explained above.

The above method may, for example, be realized as a computer program which comprises instructions which, when executed, cause the processor to perform the steps of the above method (to be executed by the medical device, in particular at its processor) which is a combination of above and below specified computer instructions and data definitions that enable computer hardware to perform computational or control functions or which is a syntactic unit that conforms to the rules of a particular programming language and that is composed of declarations and statements or instructions needed for a above and below specified function, task, or problem solution.

Furthermore, the above defined method may be part of a computer program product comprising instructions which, when executed by a processor, cause the processor to perform the steps of the above defined method. Accordingly, a computer readable data carrier storing such computer program product is disclosed.

The device and method provide an algorithm for step counting which can be used within medical devices which, due to their nature, have particularly limited space as well as power and processing capacity. Therefore, the algorithm determines the number of steps of a patient based on as little data as possible, reducing the number of sensors and measuring units required, and with as few computational operations as possible. For this, the acceleration data of an accelerometer unit which is commonly part of medical devices is used and processed by a processor, wherein the algorithm particularly uses the fixed arrangement of the medical device, i.e., inter alia, the fixed arrangement due to the implantation, to only determine the number of steps based on at least 2-dimensional acceleration data.

The patient's steps can thus be counted at least predominantly in their entirety, so that it is possible for an HCP to objectively assess the patient's physical activity and the information derived therefrom.

The present disclosure will now be described in further detail with reference to the accompanying schematic drawings, wherein
- Fig. 1: shows an embodiment of the medical device implanted within a patient's body, wherein the body is shown in a longitudinal section,
- Fig. 2: depicts the medical device of Fig. 1 a perspective side view,
- Fig. 3: shows the medical device of Fig. 1 with the ideal orientation of the axes of accelerometer with regard to the first and second orthogonal axis of the patient's body in a front view,
- Fig. 4: depicts the medical device of Fig. 1 within the patient's body in a front view, wherein the axes of the accelerometer are rotated with regard to the first and second orthogonal axis of the patient's body,
- Fig. 5: shows a flowchart of the algorithm,
- Fig. 6: depicts a diagram containing an example of acceleration data in g over time for a walking activity, and
- Fig. 7: an example of processed acceleration data (in m/s²) over time derived from the data shown in Fig. 7 eligible for step counting and identified steps.

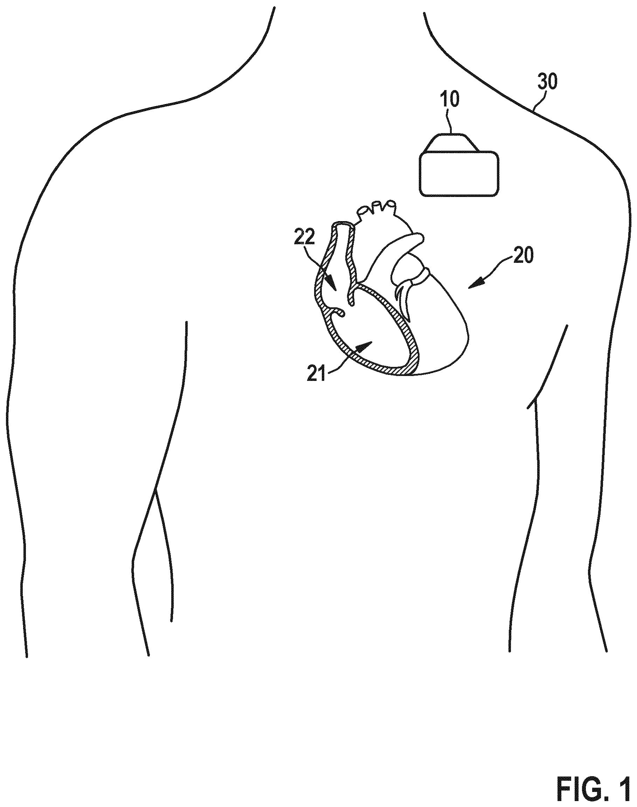

Fig. 1 shows an example medical device 10 in form of a pacemaker and a heart 20 (with right ventricle 21 and right atrium 22). The medical device 10 can also be referred to as a pacemaker 10 here. The disclosure may be realized in other medical devices analogously.

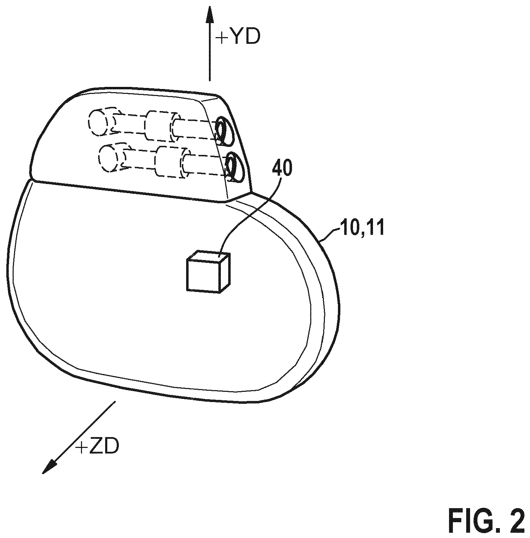

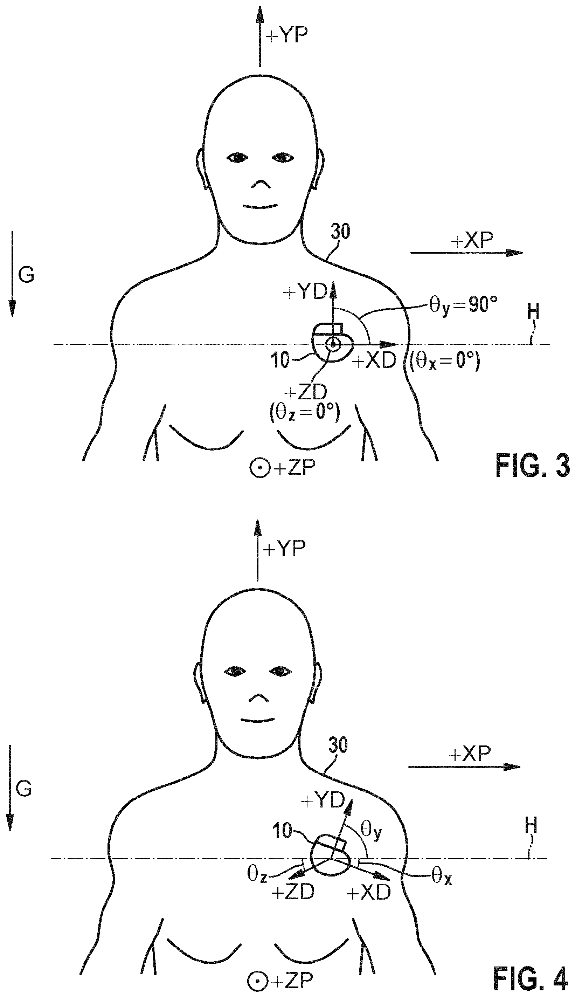

The pacemaker 10 is implanted within a patient's body 30 at its pectoral region. The pacemaker 10 may be configured to pace the right ventricle 21 and the right atrium 22 of the heart 20 in a well-known manner using electrode leads implanted in the heart 20 and connected to the pacemaker 10. The pacemaker 10 further comprises an accelerometer unit 40 shown in Fig. 2. Obviously, the medical device 10 as well as the accelerometer unit 40 comprise three spatial (orthogonal) axes, wherein, however, only two axes YD and ZD are depicted in Fig. 2. This is due to the fact that here only 2-dimensional acceleration data is needed to perform the method. Therefore, Fig. 2 only shows two exemplary axes of the accelerometer unit 40 along with the accelerometer unit gathers acceleration data. However, as will be explained with respect to Fig. 4 these axes are not necessarily parallel to the patient's body axes shown in Fig. 4. Additionally, depending on the orientation of the medical device gathering acceleration data along two alternative axes such as XD and YD is possible or providing a component of the gathered acceleration data to the processor as long as gathering said acceleration data relates to the acceleration of the patient's body 30 along the first orthogonal axis YP and the second orthogonal axis ZP depicted in Fig. 3 to 4. Further, the accelerometer unit 40 is mounted in the housing 11 of the pacemaker 10 and hermetically sealed therein. Additionally, the pacemaker 10 may comprise modules/units (not shown) such as a processor, a data memory unit, a signal generator unit for providing treatment signals (e.g. pacing signals), a sensor comprising an IEGM measuring unit for sensing ventricular depolarization, a transceiver module for sending and receiving messages to a Programmer or remote computer, and a power source wherein the modules/units are electrically connected to each other. The power source may include a battery, e.g., a rechargeable or non-rechargeable battery. The data memory unit may include any memory type mentioned above.

The accelerometer unit 40 comprises an accelerometer sensor, for example ultralow power MEMS accelerometer ADXL362 of Analog Devices, which is sensitive to both DC and AC accelerations in at least two orthogonal directions depicted in Fig. 2 with YD and ZD. The DC component of accelerations experienced by the sensor is due to the gravitational field. Assuming there is no AC component of acceleration (i.e. there is no dynamic movement) the sensor will produce specific outputs when aligned with the gravitational field in various orientations.

For a pacemaker 10 implanted in the chest of the patient, the ideal scenario from an orientation perspective is when the accelerometer's YD and ZD axes are aligned with the patient's body YP and ZP axes. The patient's body +YP axis is the patient's craniocaudal or similar axis as defined above. The patient's body +ZP axis is the patient's dorsoventral or similar axis as defined above. If the patient is in a standing (upright) position, this means that the patient's body +YP and +ZP axes are orthogonal. An +XP axis which is directed to the left arm of the patient is also shown even though the respective acceleration data of this axis are not used for step counting as described below. Further, the YP,ZP-plane is orthogonal to the ground, respectively. The XP,ZP plane defined as a horizontal plane H is orthogonal to the gravitation direction G, wherein the gravitation direction G, which is directed towards the ground, is parallel to the YP axis.

Fig. 3 illustrates the ideal orientation of the accelerometer's axes (XD, YD, ZD) and patient's body axes (XP, YP, ZP) with respect to the earth's gravitational field, if the patient is standing. In this configuration the part of the accelerometer unit of the medical device 10 which is configured to determine acceleration data along the YD axis of the accelerometer unit is in line with the YP axis of the patient. Thus, the acceleration data gathered by said accelerometer unit along its YD axis matches completely with the acceleration of the patient's body 30 along his or her first orthogonal axis YP. Due to the orientation of the pacemaker's axes, the same applies for the ZD axis of the accelerometer unit and the ZP axis of the patient. Therefore, the acceleration of the patient in his or her ZP axis, for example during walking, perfectly matches the gathered acceleration data of the accelerometer unit along its ZD axis.

Typically, due to implantation considerations or movement of the medical device in relation to the patient's body after implantation the accelerometer's and patient's body axes do not align in the ideal orientation as depicted in Fig. 4. In this figure, the pacemaker 10 (and its accelerometer) is rotated from its ideal position; specifically, it has been rotated about the patient's body XP and ZP axes, thereby taking the angles (θₓ, θ_{y}, θ_{z}) between the accelerometer's axes and the horizontal plane H.

Methods for determining the orientation of the medical devices and especially the axes XD, YD and ZD respectively within a human body are well known and may be used in order to determine the orientation of the device and its accelerometer unit, respectively. In an exemplary case, the step counting method explained below uses the acceleration components along the YP and ZP axes which may be calculated if the orientation of the accelerometer unit 40 with regard to the patient's axes XP, YP, ZP is known.

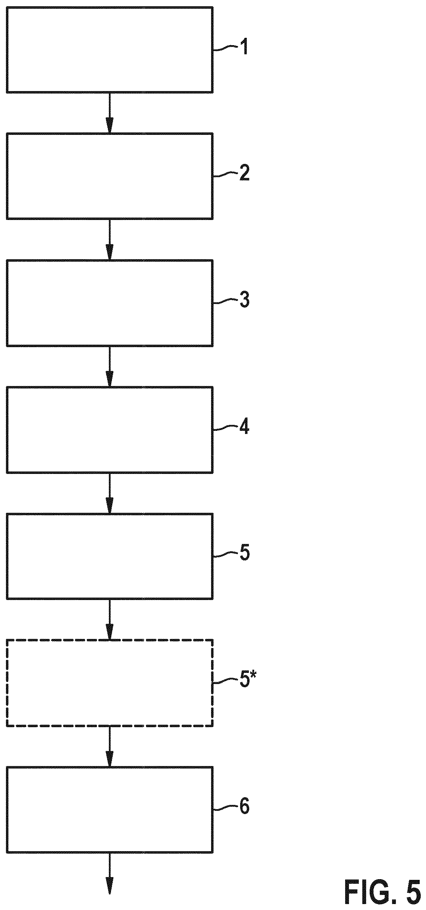

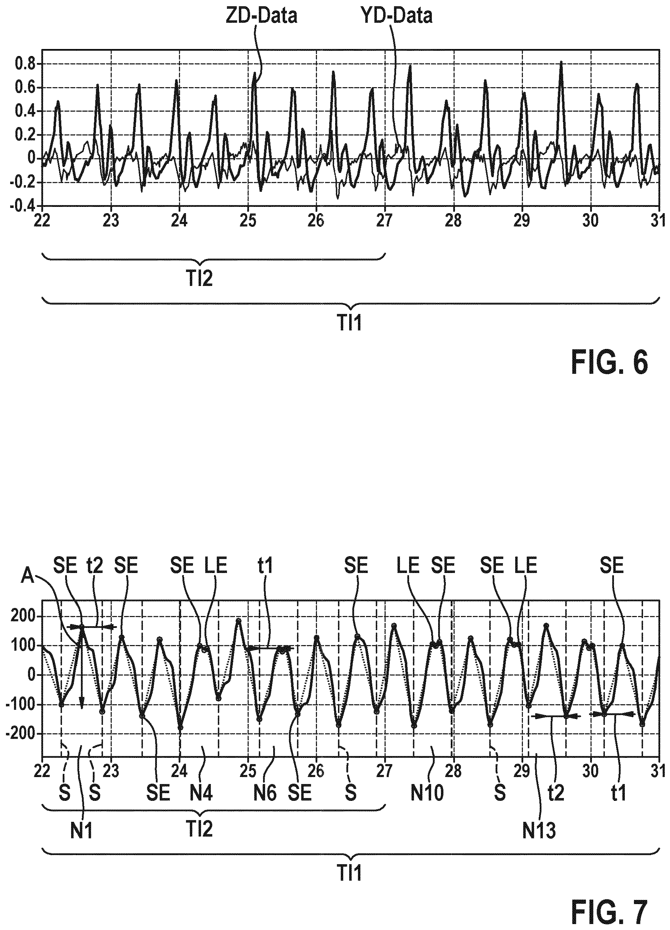

In the following it is assumed that the accelerometer axes YD and ZD of the accelerometer unit 40 of the pacemaker 10 are parallel to the patient's body axes YP and ZP. In order to determine a step count, the accelerometer unit 40 determines acceleration data YD-data and ZD-data within a first time interval TI1 and preprocesses these data (see step 1 in Fig. 5 and data depicted in Fig. 6), for example digitize these data. The first time interval TI1 in Fig. 6, 7 has a length of 9 seconds. The data YD-data and ZD-data are then transmitted to the processor of the pacemaker 10. The algorithm then proceeds to step 2, wherein the processor bandpass filters the acceleration data. To do so, the applied bandpass filter has a high pass cutoff at, for example, 0.25 Hz and a low pass cutoff at, for example, 6 Hz. In step 3, the filtered acceleration data YD-data and ZD-data are summed, wherein for each point in time the YD-Data is added to the respective ZD-data and denotes a new acceleration data at the discrete point in time. Further, in step 4 the summed acceleration data is smoothed using a moving average as described above, wherein the width of the moving average window is, for example 0.3 seconds. The solid line in Fig. 7 shows the summed and smoothed acceleration data over time. Afterwards, in step 5 the first derivative is determined and used to locate the local extrema LE of the summed and smoothed acceleration data. The local extrema LE and SE are indicated in Fig. 7 by small circles and the dashed line connects those circles. Then, in step 6 the processor determines a step count based on significant extrema SE eligible for step counting which are identified in step 5* from the local extrema LE identified in step 5. One step is counted after expiration of a step interval N1, N4, N6, N10, N13 in Fig. 7, for example. Each step interval separated by two vertical lines S in Fig. 7 from the neighboring step intervals.

Further, step 5* is shown in Fig. 5 in dashed line indicating that in order to identify the significant extrema SE out of the extrema determined in step 5 different operations (identification steps), such as a comparison of peak-to-peak amplitudes, time differences etc. with thresholds, may be performed to identify those local extrema LE which are not regarded as significant extrema SE eligible for step counting.

In order to determine the number of steps with a high accuracy identification steps as indicated above may be applied to the local extrema LE identified by the first derivative. The assessed parameters are shown in Fig. 7.

At first, the peak-to-peak amplitude A (see step interval N1) is determined between each two consecutive local extrema and is compared with a pre-defined first threshold, which is, for example 50. One of the two consecutive local extrema is not regarded as a significant extremum (SE) eligible for step counting if the determined amplitude (A) is less than or equal to the first threshold. As one can derive from the step interval N4 the first local maximum and the second local maximum have a peak-to-peak amplitude A which is less than a the first threshold, so that one of these extrema may be a significant extremum SA and one is not regarded as significant extremum but is still a local extremum LE. Then, the polarity order is determined of at least three consecutive local extrema. In the embodiment shown in Fig. 7 almost in each step interval the polarity of the local extrema is "-", "+" and "-", for example in step interval N1. For example, in the step interval N4, N6 and N10, such polarity order is not observed. Accordingly, some of the local extrema of the latter step intervals do not provide this polarity change and are therefore not regarded significant extrema SE. In the next identification step, the time difference t1, t2 between consecutive local extrema are determined, wherein t1 is the time interval between a local minimum and a local maximum and t2 the time interval between a local maximum and a local minimum. If the time difference t1, t2 of two consecutive local extrema is equal to or greater than a pre-defined second threshold, wherein the second threshold is, for example, 1 second, one of the two local extrema LE is not regarded as significant extrema SE. This not observed in the example shown in Fig. 7. The next identification step is directed to the time difference t2, only. One of the local minimum and the local maximum is not regarded as a significant minimum or significant maximum eligible for step counting, respectively, if the time difference is less than or equal to a pre-defined third threshold, wherein the third threshold is, for example 0.1 seconds. This is true, for example, for the two positive local extrema of the step interval N4. In the next identification step the peak-to-peak amplitudes A of each two consecutive of at least three consecutive extrema is determined, wherein the processor is configured to sum these determined peak-to-peak amplitudes A. At least one of the at least three consecutive extrema is not regarded as a significant extremum SE eligible for step counting if the sum is less than or equal to a pre-defined fourth threshold. The fourth threshold may have a value as indicated above.

The above identification steps are provided for each three consecutive local extrema. Then, the number of steps is counted in the first time interval TI1 using the significant extrema SE. As the local minima with a negative acceleration value (see Fig. 7) after summing and smoothing seem to have lower noise, steps are counted starting from the first significant minimum (see vertical line S on the left hand side of step interval N1) having a negative value. The next step begins with the next local minimum at a negative value (see next vertical line S. In the first time interval TI1 shown in Fig. 7 of approximately 9 seconds 15 steps are counted.

In one embodiment isolated counted steps may be discarded if they are not followed by a pre-defined number of steps (e.g. 5 steps) within a second time interval TI2, wherein the second time interval TI2 may have a duration of 5 seconds.

The resulting number of steps is the determined step number minus the discarded steps.

In another embodiment, the output of step counting method/device may be, for example, a flag for each counted step.

The above embodiment shows again, that the device and method as well as computer program product and computer readable data carrier which may be derived from above

explanation of device and method contain a practical solution with regard to integration of a simple, cost- and power-effective objective quality of life metrics in a low power wearable or implanted device.

The invention is defined by the appended claims.

### List of reference numbers

- 10: medical device
- 11: housing
- 20: heart
- 21: right ventricle
- 22: right atrium
- 30: patient's body
- 40: accelerometer unit

- A: peak-to-peak amplitude
- G: gravitation direction
- H: horizontal plane
- LE: local extremum
- N1, N4, N6, N10, N13: step interval
- S: vertical line
- SE: significant extremum
- t1: time difference
- t2: time difference
- TI1: first time interval
- TI2: second time interval
- XP: third orthogonal axis of the patient
- YP: first orthogonal axis of the patient
- ZP: second orthogonal axis of the patient
- XD: axis of accelerometer unit
- YD: axis of accelerometer unit
- ZD: axis of accelerometer unit
- YD-data: data acceleration data related to the first orthogonal axis
- ZD-data: data acceleration data related to the second orthogonal axis

## Claims

1. A medical device (10) for a patient's body (30) comprising an accelerometer unit (40) and a processor which are electrically interconnected, wherein the patient's body has two orthogonal axes (YP, ZP), wherein the first orthogonal axis (YP) is given by the craniocaudal axis of the patient's body and the second orthogonal axis (ZP) is given by the dorsoventral axis of the patient's body, wherein the accelerometer unit is configured to determine at least 2-dimensional acceleration data (YD-data, ZD-data) over time along the first orthogonal axis (YP) and the second orthogonal axis (ZP), and wherein the processor is configured
• to receive a group of said acceleration data (YD-data, ZD-data) determined by the accelerometer unit within a predefined first time interval (TI1),
• to apply a bandpass filter to said acceleration data, the bandpass filter comprising a high pass cut off in the range of 0.001 Hz to 0.5 Hz and a low pass cut off in the range of 3 Hz to 6 Hz,
• to apply the following two steps to said filtered acceleration data in any order
▪ summing said filtered acceleration data of the first orthogonal axis and the second orthogonal axis discretely for each point in time,
▪ smoothing said filtered acceleration data by calculating a moving average using a moving average window of a pre-defined width,
• to identify local extrema (LE) of the smoothed and summed acceleration data based on the first derivative of these acceleration data,
• to identify significant extrema (SE) eligible for step counting based on at least one pre-defined condition from the local extrema (LE), and
• to determine a step count based on the significant extrema (SE) within said pre-defined first time interval.

2. The medical device of claim 1, wherein the width of the moving average window is between 0.1 seconds and 0.6 seconds.

3. The medical device of any of the previous claims, wherein the processor is configured to determine a peak-to-peak amplitude (A) of two consecutive local extrema (LE) or significant extrema (SE), wherein one of the two consecutive local extrema is not regarded as a significant extremum (SE) eligible for step counting if the determined amplitude (A) is less than or equal to a pre-defined first threshold.

4. The medical device of any of the previous claims, wherein the processor is configured to determine the polarity of two consecutive local extrema (LE) or significant extrema (SE), and wherein one of the two extrema is not regarded as a significant extremum (SE) eligible for step counting if the acceleration data (YD-data, ZD-data) of these extrema have the same polarity, wherein the polarity of an extremum refers to the algebraic sign of the respective acceleration value.

5. The medical device of any of the previous claims, wherein the processor is configured to determine a time difference (t1) between two consecutive local (LE) or significant extrema (SE), and wherein one of the two extrema is not regarded as a significant extremum (SE) eligible for step counting if the determined time difference (t1) is greater than or equal to a pre-defined second threshold.

6. The medical device of any of the previous claims, wherein the processor is configured to determine a time difference (t2) between a local maximum or significant maximum and a consecutive local minimum or significant minimum, and wherein one of the minimum and the maximum is not regarded as a significant minimum or significant maximum eligible for step counting, respectively, if the time difference is less than or equal to a pre-defined third threshold.

7. The medical device of any of the previous claims, wherein the processor is configured to determine the peak-to-peak amplitudes (A) of each two consecutive of at least three consecutive extrema, wherein the processor is configured to sum said determined peak-to-peak amplitudes, wherein at least one of the at least three consecutive extrema is not regarded as a significant extremum (SE) eligible for step counting if the sum is less than or equal to a pre-defined fourth threshold.

8. The medical device of any of the previous claims, wherein the processor is configured to discard a counted step if it is not preceded by a pre-defined first number of steps and/or followed by a pre-defined second number of steps within a predefined second time interval (TI2).

9. A method for determining the number of steps made by a patient using a medical device (10) for a patient's body (30) comprising an accelerometer unit (40) and a processor which are electrically interconnected, wherein the patient's body has two orthogonal axes (YP, ZP) , wherein the first orthogonal axis (YP) is given by the craniocaudal axis of the patient's body and the second orthogonal axis (ZP) is given by the dorso-ventral axis of the patient's body, wherein the accelerometer unit determines at least 2-dimensional acceleration data (YD-data, ZD-data) over time along the first orthogonal axis (YP) and the second orthogonal axis (ZP), and wherein the processor
• receives a group of said acceleration data (YD-data, ZD-data) determined by the accelerometer unit within a predefined first time interval (TI1),
• applies a bandpass filter to said acceleration data, the bandpass filter comprising a high pass cut off in the range of 0.001 Hz to 0.5 Hz and a low pass cut off in the range of 3 Hz to 6 Hz,
• applies the following two steps to said filtered acceleration data in any order
▪ summing said filtered acceleration data of the first orthogonal axis and the second orthogonal axis discretely for each point in time,
▪ smoothing said filtered acceleration data by calculating a moving average using a moving average window of a pre-defined width, the width is, for example, between 0.1 and 0.6 seconds,
• identifies local extrema (LE) of the smoothed and summed acceleration data based on the first derivative of these acceleration data,
• identifies significant extrema (SE) eligible for step counting based on pre-defined conditions from the local extrema (LE), and
• determines a step count based on the significant extrema (SE) within said predefined first time interval.

10. The method of claim 9, wherein the processor determines a peak-to-peak amplitude (A) of two consecutive local extrema (LE) or significant extrema (SE), wherein one of the two consecutive local extrema or significant extrema (SE) is not regarded as a significant extremum (SE) eligible for step counting if the determined amplitude (A) is less than or equal to a pre-defined first threshold.

11. The method of any of claims 9 to 10. wherein the processor determines the polarity of two consecutive local extrema (LE) or significant extrema (SE), and wherein one of the two extrema is not regarded as a significant extremum (SE) eligible for step counting if the acceleration data (YD-data, ZD-data) of these extrema have the same polarity, wherein the polarity of an extremum refers to the algebraic sign of the respective acceleration value.

12. The method of any of claims 9 to 11, wherein the processor determines a time difference (t1) between two consecutive local (LE) or significant extrema (SE), and wherein one of the two extrema is not regarded as a significant extremum (SE) eligible for step counting if the determined time difference (t1) is greater than or equal to a pre-defined second threshold.

13. The method of any of claims 9 to 12, wherein the processor determines a time difference (t2) between a local maximum or significant maximum and a consecutive local minimum or significant minimum, and wherein one of the minimum and the maximum is not regarded as a significant minimum or significant maximum eligible for step counting, respectively, if the time difference is less than or equal to a pre-defined third threshold and/or
wherein the processor determines the peak-to-peak amplitudes (A) of each two consecutive of at least three consecutive extrema, wherein the processor sums said determined peak-to-peak amplitudes, and wherein at least one of the at least three consecutive extrema is not regarded as a significant extremum (SE) eligible for step counting if the summed amplitude is less than a pre-defined fourth threshold.

14. A computer program product comprising instructions which, when executed by a processor according to any of the claims 1 to 8, cause the processor to perform the steps of the method according to any of the claims 9 to 13.

15. Computer readable data carrier storing a computer program product according to claim 14.

## Patentansprüche

1. Medizinische Vorrichtung (10) für einen Körper eines Patienten (30), eine Beschleunigungssensoreinheit (40) und einen Prozessor umfassend, die elektrisch miteinander verbunden sind, wobei der Körper des Patienten zwei orthogonalen Achsen (YP, ZP) aufweist, wobei die erste orthogonale Achse (CP) durch die kraniokaudale Achse des Körpers des Patienten gegeben ist und die zweite orthogonale Achse (ZP) durch die dorsoventrale Achse des Körpers des Patienten gegeben ist, wobei die Beschleunigungssensoreinheit dafür konfiguriert ist, mindestens zweidimensionale Beschleunigungsdaten (YD-Daten, ZD-Daten) im Zeitverlauf entlang der ersten orthogonalen Achse (YP) und der zweiten orthogonalen Achse (ZP) zu bestimmen, und wobei der Prozessor dafür konfiguriert ist,
• eine Gruppe der genannten Beschleunigungsdaten (YD-Daten, ZD-Daten) zu empfangen, die durch die Beschleunigungssensoreinheit innerhalb eines vordefinierten ersten Zeitintervalls (TI1) bestimmt werden,
• einen Bandpassfilter auf die genannten Beschleunigungsdaten anzuwenden, wobei der Bandpassfilter einen Hochpass-Cutoff im Bereich von 0,001 Hz bis 0,5 Hz und einen Tiefpass-Cutoff im Bereich von 3 Hz bis 6 Hz umfasst,
• die folgenden zwei Schritte auf die genannten gefilterten Beschleunigungsdaten in beliebiger Reihenfolge anzuwenden,
▪ diskretes Summieren der genannten gefilterten Beschleunigungsdaten der ersten orthogonalen Achse und der zweiten orthogonalen Achse für jeden Zeitpunkt,
▪ Glätten der genannten gefilterten Beschleunigungsdaten, indem ein gleitender Durchschnitt anhand eines Fensters des gleitenden Durchschnitts von vordefinierter Breite berechnet wird,
• lokale Extremwerte (LE) der geglätteten und summierten Beschleunigungsdaten auf Grundlage der ersten Ableitung dieser Beschleunigungsdaten zu identifizieren,
• signifikante Extremwerte (SE) zu identifizieren, die für eine Schrittzählung auf Grundlage von mindestens einer vordefinierten Bedingung aus den lokalen Extremwerten (LE) infrage kommen, und
• eine Schrittzahl auf Grundlage der signifikanten Extremwerte (SE) innerhalb des genannten vordefinierten ersten Zeitintervalls zu bestimmen.

2. Medizinische Vorrichtung nach Anspruch 1, wobei die Breite des Fensters des gleitenden Durchschnitts zwischen 0,1 Sekunden und 0,6 Sekunden liegt.

3. Medizinische Vorrichtung nach einem der vorstehenden Ansprüche, wobei der Prozessor dafür konfiguriert ist, eine Spitze-Spitze-Amplitude (A) von zwei aufeinanderfolgenden lokalen Extremwerten (LE) oder signifikanten Extremwerten (SE) zu bestimmen, wobei einer der zwei aufeinanderfolgenden lokalen Extremwerte nicht als ein signifikanter Extremwert (SE) betrachtet wird, der für die Schrittzählung infrage kommt, wenn die bestimmte Amplitude (A) kleiner oder gleich einem vordefinierten ersten Schwellenwert ist.

4. Medizinische Vorrichtung nach einem der vorstehenden Ansprüche, wobei der Prozessor dafür konfiguriert ist, die Polarität von zwei aufeinanderfolgenden lokalen Extremwerten (LE) oder signifikanten Extremwerten (SE) zu bestimmen, und wobei einer der zwei Extremwerte nicht als ein signifikanter Extremwert (SE) betrachtet wird, der für die Schrittzählung infrage kommt, wenn die Beschleunigungsdaten (YD-Daten, ZD-Daten) dieser Extremwerte dieselbe Polarität haben, wobei die Polarität eines Extremwertes sich auf das Vorzeichen des jeweiligen Beschleunigungswertes bezieht.

5. Medizinische Vorrichtung nach einem der vorstehenden Ansprüche, wobei der Prozessor dafür konfiguriert ist, eine Zeitdifferenz (t1) zwischen zwei aufeinanderfolgenden lokalen (LE) oder signifikanten Extremwerten (SE) zu bestimmen, und wobei einer der zwei Extremwerte nicht als ein signifikanter Extremwert (SE) betrachtet wird, der für die Schrittzählung infrage kommt, wenn die bestimmte Zeitdifferenz (t1) größer oder gleich einem vordefinierten zweiten Schwellenwert ist.

6. Medizinische Vorrichtung nach einem der vorstehenden Ansprüche, wobei der Prozessor dafür konfiguriert ist, eine Zeitdifferenz (t2) zwischen einem lokalen Maximum oder signifikanten Maximum und einem darauffolgenden lokalen Minimum oder signifikanten Minimum zu bestimmen, und wobei eins von dem Minimum und dem Maximum nicht als ein signifikantes Minimum oder signifikantes Maximum betrachtet wird, das jeweils für die Schrittzählung infrage kommt, wenn die Zeitdifferenz kleiner oder gleich einem vordefinierten dritten Schwellenwert ist.

7. Medizinische Vorrichtung nach einem der vorstehenden Ansprüche, wobei der Prozessor dafür konfiguriert ist, die Spitze-Spitze-Amplituden (A) von jeweils zwei aufeinanderfolgenden von mindestens drei aufeinanderfolgenden Extremwerten zu bestimmen, wobei der Prozessor dafür konfiguriert ist, die genannten Spitze-Spitze-Amplituden zu summieren, wobei mindestens einer der mindestens drei aufeinanderfolgenden Extremwerte nicht als ein signifikanter Extremwert (SE) betrachtet wird, der für die Schrittzählung infrage kommt, wenn die Summe kleiner oder gleich einem vordefinierten vierten Schwellenwert ist.

8. Medizinische Vorrichtung nach einem der vorstehenden Ansprüche, wobei der Prozessor dafür konfiguriert ist, einen gezählten Schritt zu verwerfen, wenn diesem nicht eine vordefinierte erste Anzahl Schritte vorausgeht und/oder auf diesen nicht eine vordefinierte zweite Anzahl Schritte innerhalb eines vordefinierten zweiten Zeitintervalls (TI2) folgt.

9. Verfahren zum Bestimmen der Anzahl Schritte, die ein Patient macht, unter Verwendung einer medizinischen Vorrichtung (10) für einen Körper eines Patienten (30), eine Beschleunigungssensoreinheit (40) und einen Prozessor umfassend, die elektrisch miteinander verbunden sind, wobei der Körper des Patienten zwei orthogonalen Achsen (YP, ZP) aufweist, wobei die erste orthogonale Achse (CP) durch die kraniokaudale Achse des Körpers des Patienten gegeben ist und die zweite orthogonale Achse (ZP) durch die dorsoventrale Achse des Körpers des Patienten gegeben ist, wobei die Beschleunigungssensoreinheit mindestens zweidimensionale Beschleunigungsdaten (YD-Daten, ZD-Daten) im Zeitverlauf entlang der ersten orthogonalen Achse (YP) und der zweiten orthogonalen Achse (ZP) bestimmt, und wobei der Prozessor
• eine Gruppe der genannten Beschleunigungsdaten (YD-Daten, ZD-Daten) empfängt, die durch die Beschleunigungssensoreinheit innerhalb eines vordefinierten ersten Zeitintervalls (TI1) bestimmt werden,
• einen Bandpassfilter auf die genannten Beschleunigungsdaten anwendet, wobei der Bandpassfilter einen Hochpass-Cutoff im Bereich von 0,001 Hz bis 0,5 Hz und einen Tiefpass-Cutoff im Bereich von 3 Hz bis 6 Hz umfasst,
• die folgenden zwei Schritte auf die genannten gefilterten Beschleunigungsdaten in beliebiger Reihenfolge anwendet,
▪ diskretes Summieren der genannten gefilterten Beschleunigungsdaten der ersten orthogonalen Achse und der zweiten orthogonalen Achse für jeden Zeitpunkt,
▪ Glätten der genannten gefilterten Beschleunigungsdaten, indem ein gleitender Durchschnitt anhand eines Fensters des gleitenden Durchschnitts von vordefinierter Breite berechnet wird, wobei die Breite zum Beispiel zwischen 0,1 und 0,6 Sekunden liegt,
• lokale Extremwerte (LE) der geglätteten und summierten Beschleunigungsdaten auf Grundlage der ersten Ableitung dieser Beschleunigungsdaten identifiziert,
• signifikante Extremwerte (SE) identifiziert, die für die Schrittzählung auf Grundlage vordefinierter Bedingungen aus den lokalen Extremwerten (LE) infrage kommen, und
• eine Schrittzahl auf Grundlage der signifikanten Extremwerte (SE) innerhalb des genannten vordefinierten ersten Zeitintervalls bestimmt.

10. Verfahren nach Anspruch 9, wobei der Prozessor eine Spitze-Spitze-Amplitude (A) von zwei aufeinanderfolgenden lokalen Extremwerten (LE) oder signifikanten Extremwerten (SE) bestimmt, wobei einer der zwei aufeinanderfolgenden lokalen Extremwerte oder signifikanten Extremwerte (SE) nicht als ein signifikanter Extremwert (SE) betrachtet wird, der für die Schrittzählung infrage kommt, wenn die bestimmte Amplitude (A) kleiner oder gleich einem vordefinierten ersten Schwellenwert ist.

11. Verfahren nach einem der Ansprüche 9 bis 10, wobei der Prozessor die Polarität von zwei aufeinanderfolgenden lokalen Extremwerten (LE) oder signifikanten Extremwerten (SE) bestimmt, und wobei einer der zwei Extremwerte nicht als ein signifikanter Extremwert (SE) betrachtet wird, der für die Schrittzählung infrage kommt, wenn die Beschleunigungsdaten (YD-Daten, ZD-Daten) dieser Extremwerte dieselbe Polarität haben, wobei die Polarität eines Extremwertes sich auf das Vorzeichen des jeweiligen Beschleunigungswertes bezieht.

12. Verfahren nach einem der Ansprüche 9 bis 11, wobei der Prozessor eine Zeitdifferenz (t1) zwischen zwei aufeinanderfolgenden lokalen (LE) oder signifikanten Extremwerten (SE) bestimmt, und wobei einer der zwei Extremwerte nicht als ein signifikanter Extremwert (SE) betrachtet wird, der für die Schrittzählung infrage kommt, wenn die bestimmte Zeitdifferenz (t1) größer oder gleich einem vordefinierten zweiten Schwellenwert ist.

13. Verfahren nach einem der Ansprüche 9 bis 12, wobei der Prozessor eine Zeitdifferenz (t2) zwischen einem lokalen Maximum oder signifikanten Maximum und einem darauffolgenden lokalen Minimum oder signifikanten Minimum bestimmt, und wobei eins von dem Minimum und dem Maximum nicht als ein signifikantes Minimum oder signifikantes Maximum betrachtet wird, das jeweils für die Schrittzählung infrage kommt, wenn die Zeitdifferenz kleiner oder gleich einem vordefinierten dritten Schwellenwert ist, und/oder wobei der Prozessor die Spitze-Spitze-Amplituden (A) von jeweils zwei aufeinanderfolgenden von mindestens drei aufeinanderfolgenden Extremwerten bestimmt, wobei der Prozessor die genannten Spitze-Spitze-Amplituden summiert, und wobei mindestens einer der mindestens drei aufeinanderfolgenden Extremwerte nicht als ein signifikanter Extremwert (SE) betrachtet wird, der für die Schrittzählung infrage kommt, wenn die summierte Amplitude kleiner als ein vordefinierter vierter Schwellenwert ist.

14. Computerprogrammprodukt, das Anweisungen umfasst, die bei Ausführung durch einen Prozessor nach einem der Ansprüche 1 bis 8 den Prozessor veranlassen, die Schritte des Verfahrens nach einem der Ansprüche 9 bis 13 durchzuführen.

15. Computerlesbarer Datenträger, auf dem ein Computerprogrammprodukt nach Anspruch 14 gespeichert ist.

## Revendications

1. Dispositif médical (10) pour le corps d'un patient (30) comprenant une unité d'accéléromètre (40) et un processeur qui sont interconnectés électriquement, dans lequel le corps du patient a deux axes orthogonaux (YP, ZP), dans lequel le premier axe orthogonal (YP) est donné par l'axe crânio-caudal du corps du patient et le second axe orthogonal (ZP) est donné par l'axe dorso-ventral du corps du patient, dans lequel l'unité d'accéléromètre est configurée pour déterminer au moins des données d'accélération en 2 dimensions (données YD, données ZD) en fonction du temps le long du premier axe orthogonal (YP) et du second axe orthogonal (ZP), et dans lequel le processeur est configuré
• pour recevoir un groupe desdites données d'accélération (données YD, données ZD) déterminées par l'unité d'accéléromètre dans un premier intervalle de temps prédéfini (TI1),
• pour appliquer un filtre passe-bande auxdites données d'accélération, le filtre passe bande comprenant un seuil de coupure passe-haut dans la plage de 0,001 Hz à 0,5 Hz et un seuil de coupure passe-bas dans la plage de 3 Hz à 6 Hz,
• pour appliquer les deux étapes suivantes auxdites données d'accélération filtrées dans n'importe quel ordre
▪ faire la somme desdites données d'accélération filtrées du premier axe orthogonal et du second axe orthogonal de manière discrète pour chaque point du temps,
▪ lisser lesdites données d'accélération filtrées en calculant une moyenne mobile en utilisant une fenêtre de moyenne mobile d'une largeur prédéfinie,
• pour identifier les extrêmes locaux (LE) des données d'accélération lissées et sommées en se basant sur la dérivée première desdites données d'accélération,
• pour identifier des extrêmes significatifs (SE) éligibles à un comptage de pas en se basant sur au moins une condition prédéfinie à partir des extrêmes locaux (LE), et
• pour déterminer un décompte de pas en se basant sur les extrêmes significatifs (SE) dans ledit premier intervalle de temps prédéfini.

2. Dispositif médical selon la revendication 1, dans lequel la largeur de la fenêtre de moyenne mobile est entre 0,1 seconde et 0,6 seconde.

3. Dispositif médical selon l'une quelconque des revendications précédentes, dans lequel le processeur est configuré pour déterminer une amplitude crête-à-crête (A) de deux extrêmes locaux (LE) ou extrêmes significatifs (SE) consécutifs, dans lequel l'un des deux extrêmes locaux consécutifs n'est pas considéré comme un extrême significatif (SE) éligible au comptage de pas si l'amplitude (A) déterminée est inférieure ou égale à un premier seuil prédéfini.

4. Dispositif médical selon l'une quelconque des revendications précédentes, dans lequel le processeur est configuré pour déterminer la polarité de deux extrêmes locaux (LE) ou extrêmes significatifs (SE) consécutifs, et dans lequel l'un des deux extrêmes n'est pas considéré comme un extrême significatif (SE) éligible au comptage de pas si les données d'accélération (données YD, données ZD) desdits extrêmes ont la même polarité, dans lequel la polarité d'un extrême désigne le signe algébrique de la valeur d'accélération respective.

5. Dispositif médical selon l'une quelconque des revendications précédentes, dans lequel le processeur est configuré pour déterminer une différence de temps (t1) entre deux extrêmes locaux (LE) ou significatifs (SE) consécutifs, et dans lequel l'un des deux extrêmes n'est pas considéré comme un extrême significatif (SE) éligible au comptage de pas si la différence de temps (t1) déterminée est supérieure ou égale à un deuxième seuil prédéfini.

6. Dispositif médical selon l'une quelconque des revendications précédentes, dans lequel le processeur est configuré pour déterminer une différence de temps (t2) entre un maximum local ou un maximum significatif et un minimum local ou un minimum significatif consécutif, et dans lequel un parmi le minimum et le maximum n'est pas considéré comme un minimum significatif ou un maximum significatif éligible au comptage de pas, respectivement, si la différence de temps est inférieure ou égale à un troisième seuil prédéfini.

7. Dispositif médical selon l'une quelconque des revendications précédentes, dans lequel le processeur est configuré pour déterminer les amplitudes crête-à-crête (A) de chaque deux extrêmes consécutifs parmi au moins trois extrêmes consécutifs, dans lequel le processeur est configuré pour faire la somme desdites amplitudes crête-à-crête déterminées, dans lequel au moins l'un des au moins trois extrêmes consécutifs n'est pas considéré comme un extrême significatif (SE) éligible au comptage de pas si la somme est inférieure ou égale à un quatrième seuil prédéfini.

8. Dispositif médical selon l'une quelconque des revendications précédentes, dans lequel le processeur est configuré pour rejeter un pas compté s'il n'est pas précédé d'un premier nombre de pas prédéfini et/ou suivi par un second nombre de pas prédéfini dans un second intervalle de temps prédéfini (TI2).

9. Procédé de détermination du nombre de pas faits par un patient en utilisant un dispositif médical (10) pour le corps d'un patient (30) comprenant une unité d'accéléromètre (40) et un processeur qui sont interconnectés électriquement, dans lequel le corps du patient a deux axes orthogonaux (YP, ZP), dans lequel le premier axe orthogonal (YP) est donné par l'axe crânio-caudal du corps du patient et le second axe orthogonal (ZP) est donné par l'axe dorso-ventral du corps du patient, dans lequel l'unité d'accéléromètre détermine au moins des données d'accélération en 2 dimensions (données YD, données ZD) en fonction du temps le long du premier axe orthogonal (YP) et du second axe orthogonal (ZP), et dans lequel le processeur
• reçoit un groupe desdites données d'accélération (données YD, données ZD) déterminées par l'unité d'accéléromètre dans un premier intervalle de temps prédéfini (TI1),
• applique un filtre passe-bande auxdites données d'accélération, le filtre passe-bande comprenant un seuil de coupure passe-haut dans la plage de 0,001 Hz à 0,5 Hz et un seuil de coupure passe-bas dans la plage de 3 Hz à 6 Hz,
• applique les deux étapes suivantes auxdites données d'accélération filtrées dans n'importe quel ordre
▪ faire la somme desdites données d'accélération filtrées du premier axe orthogonal et du second axe orthogonal de manière discrète pour chaque point du temps,
▪ lisser lesdites données d'accélération filtrées en calculant une moyenne mobile en utilisant une fenêtre de moyenne mobile d'une largeur prédéfinie, la largeur étant par exemple entre 0,1 et 0,6 seconde,
• identifie les extrêmes locaux (LE) des données d'accélération lissées et sommées en se basant sur la dérivée première desdites données d'accélération,
• identifie des extrêmes significatifs (SE) éligibles au comptage de pas en se basant sur des conditions prédéfinies à partir des extrêmes locaux (LE), et
• détermine un décompte de pas en se basant sur les extrêmes significatifs (SE) dans ledit premier intervalle de temps prédéfini.

10. Procédé selon la revendication 9, dans lequel le processeur détermine une amplitude crête-à-crête (A) de deux extrêmes locaux (LE) ou extrêmes significatifs (SE) consécutifs, dans lequel l'un des deux extrêmes locaux ou extrêmes significatifs (SE) consécutifs n'est pas considéré comme un extrême significatif (SE) éligible au comptage de pas si l'amplitude (A) déterminée est inférieure ou égale à un premier seuil prédéfini.

11. Procédé selon l'une quelconque des revendications 9 à 10, dans lequel le processeur détermine la polarité de deux extrêmes locaux (LE) ou extrêmes significatifs (SE) consécutifs, et dans lequel l'un des deux extrêmes n'est pas considéré comme un extrême significatif (SE) éligible au comptage de pas si les données d'accélération (données YD, données ZD) de ces extrêmes ont la même polarité, dans lequel la polarité d'un extrême désigne le signe algébrique de la valeur d'accélération respective.

12. Procédé selon l'une quelconque des revendications 9 à 11, dans lequel le processeur détermine une différence de temps (t1) entre deux extrêmes locaux (LE) ou extrêmes significatifs (SE) consécutifs, et dans lequel l'un des deux extrêmes n'est pas considéré comme un extrême significatif (SE) éligible au comptage de pas si la différence de temps (t1) déterminée est supérieure ou égale à un deuxième seuil prédéfini.

13. Procédé selon l'une quelconque des revendications 9 à 12, dans lequel le processeur détermine une différence de temps (t2) entre un maximum local ou un maximum significatif et un minimum local ou un minimum significatif consécutif, et dans lequel un parmi le minimum et le maximum n'est pas considéré comme un minimum significatif ou un maximum significatif éligible au comptage de pas, respectivement, si la différence de temps est inférieure ou égale à un troisième seuil prédéfini et/ou dans lequel le processeur détermine les amplitudes crête-à-crête (A) de chaque deux extrêmes consécutifs parmi au moins trois extrêmes consécutifs, dans lequel le processeur fait la somme desdites amplitudes crête-à-crête déterminées, et dans lequel au moins l'un d'au moins trois extrêmes consécutifs n'est pas considéré comme un extrême significatif (SE) éligible au comptage de pas si l'amplitude sommée est inférieure à un quatrième seuil prédéfini.

14. Produit de programme informatique comprenant des instructions qui, lorsqu'elles sont exécutées par un processeur selon l'une quelconque des revendications 1 à 8, amènent le processeur à réaliser les étapes du procédé selon l'une quelconque des revendications 9 à 13.

15. Support de données lisible par ordinateur stockant un produit de programme informatique selon la revendication 14.
